# EUROPÄISCHE PATENTSCHRIFT

(11) **EP 3 052 435 B1**
(45) Veröffentlichungstag und Bekanntmachung des Hinweises auf die Patenterteilung: **08.11.2017**
(21) Anmeldenummer: 14776885.7
(22) Anmeldetag: 29.09.2014
(51) Int. Cl.: C01B 3/24, C01B 3/00, C07C 1/00, C25B 1/04, C25B 15/08, F01D 15/10, F01K 17/06, F02C 3/20, F02C 6/18, H01M 8/0656, H01M 8/02, H02J 15/00

(54) **VERFAHREN ZUR SPEICHERUNG VON ELEKTRISCHER ENERGIE**
METHOD FOR STORING ELECTRIC ENERGY
PROCÉDÉ DE STOCKAGE D'ÉNERGIE ÉLECTRIQUE

(30) Priorität: 30.09.2013 DE 102013219681
(43) Veröffentlichungstag der Anmeldung: 10.08.2016
(73) Patentinhaber: Fulde, Marek, 63071 Offenbach (DE)
(72) Erfinder: Fulde, Marek, 63071 Offenbach (DE)
(74) Vertreter: Jacobi, Markus Alexander
(86) Internationale Anmeldenummer: PCT/EP2014/070758
(87) Internationale Veröffentlichungsnummer: WO 2015/044407

(56) Entgegenhaltungen:
- WO-A2-2013/034130
- DE-A1-102007 037 672
- DE-A1-102012 203 334

## Beschreibung

Die Erfindung betrifft ein Verfahren zur Speicherung von elektrischer Energie durch Umwandeln von elektrischer Energie in Methangas. Des Weiteren betrifft die Erfindung ein System zum Speichern von elektrischer Energie.

Die erneuerbaren Energiequellen wie Photovoltaik oder Windenergie erlauben die Gewinnung von Energie, insbesondere von elektrischem Strom, ohne die Emission von klimaschädlichen Gasen wie Kohlendioxid (CO₂). Bei Nutzung von fossilen Energieträgern wie Öl, Kohle oder Erdgas werden hingegen große Mengen an schädlichem CO₂ emittiert. Wind und Sonne stehen jedoch naturgemäß nicht ständig zur Verfügung. Sonneneinstrahlung ist nur tagsüber verfügbar. Des Weiteren hängt die Verfügbarkeit von Sonnen- und Windenergie von den Wetterbedingungen und der Jahreszeit ab. Dies hat zur Folge, dass sich der Verbrauch bzw. der Energiebedarf selten mit der Energieproduktion aus erneuerbaren Energien deckt. Nach bisheriger Planung werden daher konventionelle Kraftwerke weiter vorgehalten, um eine zeitweilige Unterversorgung mit elektrischer Energie auszugleichen. Eine vollständige Vermeidung von CO₂-Emissionen ist so nicht möglich.

Von entscheidender Bedeutung für eine effektive Nutzung der erneuerbaren Energien ist es demnach, überschüssige elektrische Energie zu speichern, um Spitzenlasten sowie eine Unterversorgung bei ungünstigen Wetterbedingungen auszugleichen. Bisher übliche Speicher wie Pumpspeicherkraftwerke können jedoch nur geringe Energiemengen speichern und geeignete Standorte für den Aufbau weiterer Speicher sind ausgeschöpft. Neuartige Speicher für elektrische Energie sind daher Gegenstand zahlreicher Untersuchungen.

Eine Speichermöglichkeit stellen adiabatische Druckluftspeicher dar, bei denen bei einem Überangebot an elektrischer Energie Kompressoren Luft verdichten und in unterirdischen Kavernen speichern. Die Druckluft kann dann genutzt werden, um Turbinen zur Stromerzeugung anzutreiben. Nachteilig ist jedoch, dass die Druckluftspeicher nur vergleichsweise wenig elektrische Energie speichern können. Zudem sind adiabatische Druckluftspeicher nur für kurzzeitige Speicherung geeignet, da die bei der Komprimierung der Luft entstehende Wärme nicht beliebig lange gespeichert werden kann.

Die Nutzung von Wasserstoff aus der Wasserelektrolyse als Speichermedium wird ebenfalls intensiv untersucht. Wasserstoff eignet sich als emissionsfreier Brennstoff, da er zu Wasser verbrennt. Jedoch lässt sich Wasserstoff nur schwer transportieren, so dass im Fall der Speicherung von elektrischer Energie die Rückumwandlung des Wasserstoffs am gleichen Ort erfolgen sollte. Bevorzugt wäre es jedoch, die Rückumwandlung an dem Ort durchzuführen, an dem die elektrische Energie gerade benötigt wird. Insbesondere bei Offshore-Windenergieanlagen fallen der Ort der Energieerzeugung und der Ort, an dem die Energie benötigt wird, weit auseinander, was die Stromnetze stark belastet.

Eine weitere Speichermöglichkeit stellt die Erzeugung von Methangas dar. Dabei wird in einer Sabatier-Reaktion aus Wasserstoff und Kohlenstoff Methangas hergestellt. Zur Speicherung und zum Transport kann das Methangas in das bestehende Erdgasnetz eingeleitet werden, da Methan auch Hauptbestandteil von Erdgas ist. Auf diese Weise könnte die Energie nicht nur gespeichert, sondern auch ohne zusätzliche Belastung der Stromnetze vom Energieerzeugungsort zu dem Ort transportiert werden, an dem die elektrische Energie benötigt wird.

Aus DE-A 10 2007 037 672 ist ein Verfahren bekannt, bei dem regenerativ erzeugte Energie in Wasserstoff umgewandelt und anschließend zusammen mit CO₂ in Methan umgesetzt wird. Das für die Reaktion notwendige CO₂ wird aus den Abgasen von Kraftwerken abgeschieden. Das erzeugte Methan kann anschließend zur Stromerzeugung in einem Kraftwerk verbrannt werden, wobei das entstehende CO₂ wiederum abgeschieden wird, so dass ein CO₂ Kreislauf entsteht.

Aus WO 2013/034130 A2 ist ein Verfahren zur Nutzung von Energie aus Biomasse bekannt, wobei in einer ersten Betriebsphase ein Gemenge aus Biomasse und fossilem Kohlenstoff wie beispielsweise Kohle in Synthesegas überführt wird. Das Synthesegas wird in einem Gaskraftwerk verstromt und das dabei gebildete Kohlendioxid wird im Boden gespeichert oder in einer zweiten Betriebsphase Synthesegas mit aus überschüssiger elektrischer Energie durch Wasserelektrolyse hergestellten Wasserstoff in Methan umgesetzt. Das Methan wird im Gasnetz gespeichert.

Aus DE 10 2012 203 334 A1 ist ein Verfahren zum Betreiben einer Power-to-Gas-Einrichtung bekannt. Dabei wird elektrische Energie verwendet, um Wasserstoff mittels Elektrolyte zu erzeugen und zusammen mit CO₂ zu Methan umzusetzen, welches in ein Erdgasnetz eingespeist werden kann. Das CO₂ wird dazu entweder aus der Luft entnommen oder aus einem CO2-Tank zur Verfügung gestellt. Ferner ist vorgesehen, den Betrieb der Power-to-Gas-Einheit sofort zu unterbrechen und diese vom Stromnetz zu trennen, wenn die Netzfrequenz des Stromnetzes unter einen vorgegebenen Grenzwert fällt. Dadurch kann das Stromnetz so-fort entlastet werden.

Das Dokument DE 10 2009 045 564 A1 beschreibt eine Entschwefelungsanlage, bei der gleichzeitig eine Hydrodesulfurierung (HDS) und Cetanzahlanhebung von Naphtha erfolgt. Ausgangsprodukt für konventionelle Flüssigkraftstoffe ist Erdöl, welches durch Destillation in verschiedene Siedefraktionen getrennt wird. Nach der atmosphärischen Destillation folgt als zweiter Prozessschritt in der Raffinerie die Entschwefelung. Zur Entfernung der Schwefelverbindungen wird die Hydrodesulfurierung eingesetzt, bei der das Erdöldestillat über einen festen, feinteiligen Katalysator geleitet wird, der ein Metall zur Hydrierung aufweist, der von einer Aluminiumoxid-Schichtunterlage getragen wird. Als Katalysatoren dienen Nickel-Molybdän- und Chrom-Molybdän-Kontakte. Darüber hinaus werden reichliche Mengen von Wasserstoff in die Beschickung mit einbezogen. Die am Katalysator ablaufende Hydrierungsreaktion führt zu H₂S und dem hydrierten Kohlenwasserstoffrest. Durch die Hydrodesulferierung wird Wasserstoff in dem flüssigen konventionellen Kraftstoff anstelle des Schwefels in Form von hydrierten Kohlenwasserstoffresten gebunden. Auf diese Weise kann der Wasserstoff einfach transportiert werden und trägt bei einer nachfolgenden Verbrennung des Kraftstoffs zur Energieerzeugung bei.

In dem Artikel "Hydrothreated Vegetable Oil (HVO) as a Renewable Diesel Fuel: Trade-off between NOx, Particulate Emission, and Fuel Consumption of a Heavy Duty Engine" von H. Aatola, M. Larmi, T. Sarjovaara und S. Mikkonen, 2008 SAE International Study, 2008-01-2500 wird die Verwendung von Kraftstoffen beschrieben, die über eine Hydrierung von Pflanzenölen gewonnen wurden. Bei der Hydrierung von Pflanzenölen werden ungesättigte Pflanzenöle und Wasserstoff in einen für die Verwendung in herkömmlichen Verbrennungsmotoren geeignete Kraftstoffe überführt, wobei der Wasserstoff in dem Kraftstoff gebunden wird und bei der Verbrennung des Kraftstoffs zur Energieerzeugung bei.

Nachteilig am Stand der Technik ist insbesondere, dass zum einen nur ein Teil des entstehenden CO₂ aus dem Verbrennungsabgas abgeschieden werden kann, so dass die CO₂ Emissionen zwar reduziert, nicht jedoch vollständig vermieden werden können. Zum anderen sind die Abscheidung und auch die Handhabung des gasförmigen CO₂ aufwendig.

Eine Aufgabe der vorliegenden Erfindung kann darin gesehen werden, die Handhabung des Kohlenstoffs im Kreislauf zu vereinfachen und CO₂ Emissionen zu vermeiden.

Es wird ein Verfahren zur Erzeugung von Methan unter Verwendung von elektrischer Energie und nachfolgender Energieerzeugung vorgeschlagen, umfassend die Schritte
a) Erzeugung von Methan aus Wasser und Ruß unter Verwendung von elektrischer Energie,
b) Speichern des Methans,
c) Spalten des Methans in Wasserstoff und Ruß, wobei der Wasserstoff zur Energieerzeugung verwendet wird,
wobei der bei der Methanspaltung gemäß Schritt c) anfallende Ruß aufgefangen wird und beim erneuten Durchlaufen des Verfahrens für die Methanerzeugung in Schritt a) verwendet wird, so dass ein geschlossener Kohlenstoffkreislauf entsteht.

Als Alternative zur Spaltung des Methans kann im Schritt c) eine Energieerzeugung durch Umsetzen des Methans in Ruß und Wasser in einem zyklischen Bromierungs-Oxidations-Prozess erfolgen. In diesem Fall wird dann der bei dem zyklischen Bromierungs-Oxidations-Prozess anfallende Ruß aufgefangen und beim erneuten Durchlaufen des Verfahrens für die Methanerzeugung in Schritt a) verwendet. Auch in diesem Fall entsteht ein geschlossener Kohlenstoffkreislauf.

Der Kohlenstoff (Ruß) wird bei dem vorgeschlagenen Verfahren somit nicht als Brennstoff zur Energieerzeugung verwendet, sondern dient als Träger für den Wasserstoff. Als Energieerzeugung werden hierbei sowohl die Stromerzeugung als auch die Verwendung des durch Spaltung des Methans gemäß Schritt c) gewonnenen Wasserstoffs für andere Energieanwendungen wie Heizung, Kühlung oder zum Betrieb von Transportmitteln wie Autos, LKW, Bahnen oder Schiffe angesehen. Insbesondere können mit der erzeugten Energie Gebäude geheizt oder gekühlt werden.

Bevorzugt wird das Methan in einer Strom-Methan-Umwandlungsanlage mithilfe einer Sabatier-Reaktion erzeugt. Dazu wird im ersten Schritt a) des Verfahrens bei Verfügbarkeit von elektrischer Energie Wasser (H₂O) mittels Elektrolyse in Wasserstoff (H₂) und Sauerstoff (O₂) aufgespalten. Das Wasser wird dabei vor der Elektrolyse bevorzugt mit Hilfe von Prozessdampf auf ca. 90°C erwärmt. Alternativ kann hier die Hochtemperatur-Dampfelektrolyse (HotElly) zur Anwendung kommen, bei der Wasserdampf bei Temperaturen von 900 bis 1000 °C in H₂ und O₂ gespalten wird. Die Elektrolyseprodukte Wasserstoff und Sauerstoff werden dabei zunächst in Pufferbehältern gespeichert.

Nachdem für den Betrieb einer Methanisierungs-Anlage ausreichende Mengen an Wasserstoff und Sauerstoff erzeugt wurden, wird mit der Methanisierung begonnen. Hierzu wird Ruß aus einem Lager entnommen, in einem Trockner vorgetrocknet und zusammen mit den Elektrolyseprodukten in Methan umgesetzt.

Alternativ zu Methanherstellung könnten auch andere Hydrierungsreaktionen, die die Herstellung von Kohlenwasserstoffverbindungen wie z. B. Fischer-Tropsch-Reaktion ermöglichen, Anwendung finden. Auch diese Stoffe können durch Thermospaltung in Wasserstoff und Ruß zerlegt werden.

In einer Ausführungsvariante des Verfahrens wird der Ruß nach der Trocknung zu Kohlendioxid (CO₂) verbrannt. Hierbei wird bevorzugt der bei der Elektrolyse angefallene Sauerstoff verwendet. Wird der angefallene Sauerstoff bei der Verbrennung genutzt, wird die Effektivität des Verfahrens gegenüber dem Einsatz von Luft gesteigert, da es dann keiner Abtrennung des in der Luft enthaltenen Stickstoffs vom erzeugten Methan bedarf. Die bei der Verbrennung in Form von Wärme freiwerdende Energie kann mit einem Dampferzeuger aus dem CO₂ -Gas entnommen werden.

Der Dampf kann als Prozessdampf und/oder zur Stromerzeugung verwendet werden. Dem erzeugten CO₂ wird nun der bei der Elektrolyse gewonnene Wasserstoff zugegeben, wobei ein für die Sabatier-Reaktion optimales Verhältnis zwischen Kohlendioxid und Wasserstoff eingestellt wird. Optimal ist hierbei ein stöchiometrisches (1:4) bis leicht überstöchiometrisches Verhältnis bezogen auf Wasserstoff. Das Gasgemisch strömt nun in eine Hydrierungsanlage ein, wo es auf einem Katalysator bei erhöhtem Druck im Bereich von ca. 8 bis 30 bar (0,8 bis 3,0 MPa), bevorzugt im Bereich von ca. 8 bis 10 bar zu Methan umgesetzt wird. Die bei der Sabatier-Reaktion CO₂ + 4H₂ = CH₄ + 2H₂O freiwerdende Wärme kann ebenfalls mit einem Dampferzeuger entnommen werden und als Prozessdampf genutzt werden.

In einer weiteren Ausführungsform des Verfahrens wird der Ruß nach der Trocknung einer Vergasungsanlage zugeführt. Dort wird er bei einer Temperatur zwischen 900°C und 1800 °C, bevorzugt zwischen 1200 und 1800 °C, unter Einwirkung von Sauerstoff und Wasserdampf in Synthesegas umgesetzt. Besonders bevorzugt beträgt die Temperatur bei der Umsetzung ca. 1500°C. Die bei der Reaktion des Rußes zum Synthesegas freiwerdende Wärme kann mit einem Dampferzeuger entnommen werden und als Prozessdampf genutzt werden. Anschließend wird dem Synthesegas der Wasserstoff aus der Elektrolyse zugegeben, wobei ein für die Hydrierungsreaktion optimales Verhältnis zwischen Kohlenmonoxid aus dem Synthesegas und Wasserstoff eingestellt wird. Optimal ist hierbei ein stöchiometrisches (1:3) bis leicht überstöchiometrisches Verhältnis bezogen auf Wasserstoff. Das Gasgemisch strömt nun in eine Hydrierungsanlage ein, wo das Synthesegas auf einem Katalysator in Methan umgesetzt wird, wobei CO + 3H₂ zu CH₄ + H₂O reagiert. Wiederum kann die bei der Hydrierungsreaktion freiwerdende Wärme mit einem Dampferzeuger entnommen werden und als Prozessdampf genutzt werden.

Bei der beschriebenen Verbrennung oder Teiloxidation des Rußes werden relativ hohe Temperaturen von 900°C bis zu 1800°C erreicht, um die Herstellung von schädlichen Nebenprodukten wie Teer oder höher-kettige Kohlenwasserstoffe zu vermeiden. Die hohe Enthalpie des Gasstroms lässt sich nur erschwert und unter erheblichen Verlusten durch Dampferzeugung wiedergewinnen. Alternativ wird eine Hydrierung von Ruß mit Wasserstoff bei Zusatz von Wasserdampf als Vorstufe zur Methanisierungsreaktion angewandt. In dieser Ausführungsform wird Ruß, Wasserstoff und Wasserdampf in ein fluidisiertes Feststoffbett eingeleitet. Bei Temperaturen im Bereich von 600-900°C und bei einem Druck im Bereich von 20 bis 40 bar (2 bis 4 MPa) entsteht eine Mischung von Methan, Kohlenmonoxid und Kohlendioxid. Da diese Reaktion endotherm ist, wird in einer parallel verlaufenden Reaktionsstufe das Feststoffbett durch Verbrennen des nicht umgesetzten Rußes auf die Betriebstemperatur gebracht. Hierfür wird Sauerstoff aus der Wasser-Elektrolyse verwendet. Verbrennungsgase werden der Methan-Kohlenstoffoxide-Mischung beigemischt und auf eine Temperatur im Bereich von 200 bis 250°C abgekühlt.

Die abgeführte Wärme kann zur Herstellung elektrischen Stroms verwendet werden. In der folgenden Reaktionsstufe wird wie bei anderen Verfahrensbeispielen die Methaniserung der verbleibenden Kohlenstoffoxide durchgeführt. Auch hier wird das stöchiometrische Verhältnis durch Zugabe des durch Elektrolyse gewonnenen Wasserstoffs eingestellt. Die Abwärme der exothermen Methanisierungsreaktion wird zur Stromerzeugung oder als Fernwärme benutzt.

Das erzeugte Methan kann noch Kohlendioxidreste enthalten. Diese werden in einer Kohlendioxid-Abtrennvorrichtung abgetrennt und der Methanisierungsreaktion erneut zugeführt. Zur Abtrennung des Kohlendioxids können Polyimid-Hohlfaser-Membranen verwendet werden. Das Methangas wird nun getrocknet und komprimiert. Des Weiteren wird das Methangas eingestellt, so dass der für die Einspeisung in das Gasnetz zulässige Anteil an Wasserstoff (H₂) eingehalten wird. Anschließend kann das Methangas zur Speicherung gemäß Schritt b) des Verfahrens in das öffentliche Erdgasnetz eingespeist werden. Alternativ ist es auch denkbar, das Methangas in einem Druckbehälter zu speichern.

Wird Energie benötigt, kann das erzeugte Methangas gemäß Schritt c) des Verfahrens zur Energieerzeugung in einer Methan-Strom-Umwandlungsanlage verwendet werden. Dazu wird das Methangas zunächst in einem Wärmetauscher mit Hilfe von Prozesswärme vorgewärmt. Anschließend wird das Methangas einem Plasma-Wasserstoffgenerator zugeführt. Im Plasma-Wasserstoffgenerator wird eine Spaltung des Methans in Kohlenstoff (Ruß) und in gasförmigen Wasserstoff durchgeführt. Das Plasma wird beispielsweise durch Einstrahlen von Mikrowellen erzeugt. Die Plasma-Spaltung des Methans findet bei relativ niedrigen Temperaturen zwischen ca. 400 bis 600 °C statt, so dass thermische Verluste minimiert werden. In einem dem Plasma-Wasserstoffgenerator nachgeschalteten Filter wird der Ruß vom Wasserstoff getrennt. Die Umsetzung des Methans in Wasserstoff und Ruß erfolgt mit einer Ausbeute von ca. 96 bis 97%. Auch andere für die Erzeugung von Wasserstoff aus Methan geeignete Verfahren wie z.B. das "Channel-Black-Verfahren" oder das "Degussa-Black-Verfahren" (dem Fachmann auch bekannt als Gas-Ruß-Degussa-Verfahren) können angewendet werden, obgleich diese mit einer geringeren Effektivität arbeiten. Bei diesen Verfahren wird das Methangas thermisch gespalten. Dazu wird ein Methan-Gas/Luft-Gemisch einem Brenner, bestehend aus mehreren kleinen Brennerhütchen zugeführt.

Die dort entstehenden kleinen Flammen schlagen gegen eine wassergekühlte rotierende Walze, wo ein Teil des Rußes abgeschieden wird. Der Rest wird in einer nachgeschalteten Filteranlage von der Gas-Phase abgetrennt. Die weitere Rußbehandlung bzw. Nutzung des Luft/Wasserstoff-Gemisches wird analog zum bereits beschriebenen Plasma-Verfahren vorgenommen.

Alternativ kann zur Energieerzeugung aus dem Methan eine Methan-Strom-Umwandlungsanlage verwendet werden, die auf Basis eines zyklischen Bromierungs-Oxidations-Prozesses arbeitet. Der Prozess umfasst zwei exotherme Reaktionsschritte. Dabei wird in einem ersten Schritt Methan mit Brom unter Abspaltung von Ruß in Bromwasserstoff umgesetzt. In einem zweiten Schritt wird Bromwasserstoff zu Wasser oxidiert, wobei das Brom wieder frei wird und wiederverwendet wird.

Der abgetrennte Ruß wird in einer Granulationsvorrichtung agglomeriert und abgekühlt. Als Granulationsvorrichtung kann beispielsweise ein Trommel-Granulator eingesetzt werden. Ein nachgeschalteter Trockner trocknet den Ruß, bevor dieser in einem Speicher gelagert wird. Aus dem Speicher kann der Ruß anschließend auf ein geeignetes Transportmittel verladen werden, um zur Strom-Methan-Umwandlungsanlage zurückgeführt zu werden. Der Ruß kann dann beim erneuten Durchlaufen des Verfahrens zur Erzeugung von Methan verwendet werden.

Der vom Methan abgespaltene Wasserstoff kann nun zur Energieerzeugung genutzt werden. Dazu kann der Wasserstoff mit Verbrennungsluft gemischt und in einer Gasturbine verbrannt werden. Die Gasturbine treibt einen Stromgenerator an, mit dem elektrischer Strom erzeugt wird. Die Abgase aus der Verbrennung können über einen Dampferzeuger zur Erzeugung von Prozessdampf eingesetzt werden. Der Prozessdampf kann dann zur Trocknung des Rußes und/oder zum Vorwärmen des Methangases genutzt werden.

Denkbar ist es auch, den Wasserstoff über eine Brennstoffzelle in elektrischen Strom umzusetzen, oder den Wasserstoff für andere Zwecke, beispielsweise zum Betanken von wasserstoffbetriebenen Fahrzeugen oder zum Heizen einzusetzen. In einer bevorzugten Ausführungsform des Verfahrens sind die Strom-Methan-Umwandlungsanlage und die Methan-Strom-Umwandlungsanlage räumlich voneinander getrennt, wobei das Methan über das öffentliche Gasnetz transportiert wird. Auf diese Weise ist es möglich, das Methangas in der Nähe der Energieerzeuger herzustellen und die Umwandlung des Methangases in elektrischen Strom in der Nähe der Energieabnehmer vorzunehmen.

In einer weiteren Ausführungsform der Erfindung wird bei der Methanerzeugung gemäß Schritt a) des Verfahrens oder bei der Energieerzeugung gemäß Schritt c) des Verfahrens anfallende Wärme zumindest teilweise in ein Fernwärmenetz eingespeist.

Durch die Nutzung der Wärme in Form von Kraft-Wärme-Kopplung kann der Wirkungsgrad der Anlagen erhöht werden.

Bevorzugt wird der bei der Strom-Methan-Umwandlung erforderliche elektrische Strom regenerativ erzeugt. Insbesondere eignet sich elektrischer Strom aus Windkraft und Solaranlagen, da bei diesen Anlagen die Produktion abhängig von den Wetterbedingungen und der Jahreszeit ist und nicht an den Bedarf der Energieabnehmer angepasst ist. Überschüssige Energie, die zurzeit nicht abgenommen werden kann, kann dann zur Erzeugung von Methan genutzt werden.

Des Weiteren wird ein System vorgeschlagen umfassend eine Strom-Methan-Umwandlungsanlage, wobei elektrischer Strom zusammen mit Ruß und Wasser in Methan umgewandelt wird, sowie eine Methan-Strom-Umwandlungsanlage, wobei Methan unter Abspaltung von Ruß in Wasserstoff und anschließend in elektrischen Strom umgesetzt wird, wobei Mittel vorgesehen sind, um den Ruß aus der Methan-Strom-Umwandlungsanlage zur Strom-Methan-Umwandlungsanlage zurückzuführen.

Bei der Methan-Strom-Umwandlungsanlage sind dazu Einrichtungen zum Auffangen, Agglomerieren und Trocknen des Rußes vorgesehen. Der Ruß wird in einem Speicher zwischengelagert und kann von dort in ein Transportmittel wie einen LKW oder einen Eisenbahnwagon verladen werden. Bei der Strom-Methan-Umwandlungsanlage sind Einrichtungen vorgesehen, um den Ruß aus dem Transportmittel in einen Speicher zu füllen.

Vom Speicher aus kann dann der Ruß zur Herstellung von Methan verwendet werden. Die Strom-Methan-Umwandlungsanlage sowie die Methan-Strom-Umwandlungsanlage führen dabei das beschriebene Verfahren aus, so dass der Kohlenstoff in einem Kreislauf geführt wird. Dabei wird der Kohlenstoff in fester Form zurückgeführt, was eine einfache Handhabung ermöglicht.

### Vorteile der Erfindung

Mit dem erfindungsgemäßen Verfahren wird Kohlenstoff nicht als Energiequelle verwendet, sondern als Träger für Wasserstoff. Der Kohlenstoff wird dabei einmalig dem Verfahren zugeführt und wird anschließend im Kreis geführt. Emissionen von CO₂ oder anderen Klimagasen erfolgen nicht.

Der für die Energieerzeugung benötigte Wasserstoff wird beim vorgeschlagenen Verfahren mit Hilfe von Strom aus erneuerbaren Energiequellen wie Windkraft und Sonnenenergie gewonnen. Diese liefern bei Verfügbarkeit von Wind bzw. Sonne bereits heute große Mengen an elektrischer Energie, die jedoch häufig nicht verwendet bzw. "verbraucht" werden kann. Diese überschüssige Energie kann nun mit dem vorgeschlagenen Verfahren in Methan umgesetzt werden, wobei das Methangas im öffentlichen Erdgasnetz gespeichert wird. Das Erdgasnetz kann bereits bei geringfügiger Druckerhöhung große Mengen an Gas aufnehmen. Über das bloße Speichern des Energieträgers Methan hinaus erlaubt das Erdgasnetz zudem einen Transport über große Strecken hinweg, so dass die Rückumwandlung von Methangas in elektrischen Strom in der Nähe der Verbraucher erfolgen kann. Dies entlastet die Stromnetze erheblich, da ein Großteil der erneuerbaren Energien, z.B. über Offshore Windkraftwerke, weit weg von den Verbrauchern der Energie erzeugt wird.

Bei Bedarf kann mit dem vorgeschlagenen Verfahren die gespeicherte Energie auch in Form von Wasserstoff entnommen werden, der beispielsweise zum Betanken von wasserstoffbetriebenen Fahrzeugen genutzt werden kann.

Gegenüber den bekannten Verfahren zur Umwandlung und Nutzung von Methan aus erneuerbaren Energien wird der Kohlenstoff in einem einfach zu handhabenden Kreislauf geführt. Der bei der Methanspaltung anfallende Ruß kann ohne großen Aufwand vollständig vom Wasserstroff abgetrennt und gelagert werden. Auch der Transport und die Handhabung von festem Kohlenstoff sind mit weniger Aufwand verbunden als die Handhabung von gasförmigen CO₂.

Anhand der nachstehenden Zeichnungen, der Bezugszeichenliste und der Patentansprüche wird die Erfindung eingehender beschrieben.

Es zeigen:
- Figur 1: ein Schema des Verfahrens zur Speicherung von elektrischer Energie,
- Figur 2: ein Schema einer ersten Ausführungsform einer Strom-Methan-Umwandlungsanlage,
- Figur 3: ein Schema einer zweiten Ausführungsform einer Strom-Methan-Umwandlungsanlage,
- Figur 4: ein Schema einer dritten Ausführungsform einer Strom-Methan-Umwandlungsanlage und
- Figur 5: ein Schema einer Methan-Strom-Umwandlungsanlage.

### Ausführungsformen der Erfindung

In der nachfolgenden Beschreibung der Ausführungsbeispiele der Erfindung werden gleiche oder ähnliche Komponenten und Elemente mit gleichen oder ähnlichen Bezugszeichen bezeichnet, wobei auf eine wiederholte Beschreibung dieser Komponenten oder Elemente in Einzelfällen verzichtet wird. Die Figuren stellen den Gegenstand der Erfindung nur schematisch dar.

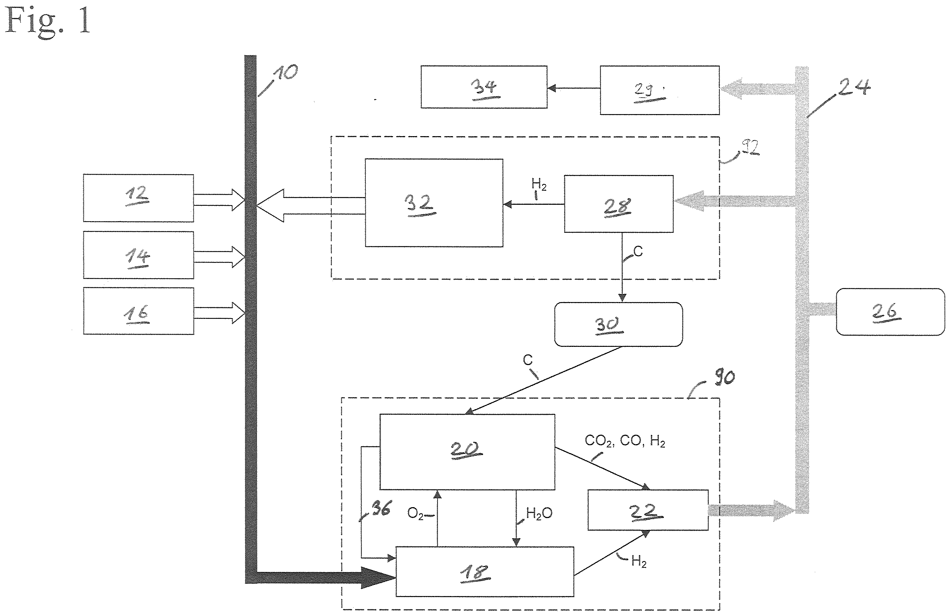

Figur 1 zeigt ein Schema des vorgeschlagenen Verfahrens zur Speicherung von elektrischer Energie.

In Figur 1 sind eine Strom-Methan-Umwandlungsanlage 90 und eine Methan-Strom-Umwandlungsanlage 92 schematisch dargestellt. Die Strom-Methan-Umwandlungsanlage 90 entnimmt elektrische Energie aus dem Stromnetz 10, sofern ein Überangebot an elektrischem Strom aus erneuerbaren Energiequellen besteht. Die erneuerbare Energie kann insbesondere Windenergie 12 oder Solarenergie 14 sein, aber auch aus sonstigen Quellen 16 wie z.B. Wasserkraft stammen.

Der elektrische Strom wird in der Strom-Methan-Umwandlungsanlage 90 genutzt, um mittels Elektrolyse 18 Wasser H₂O in Wasserstoff H₂ und Sauerstoff O₂ aufzuspalten. Der Sauerstoff O₂ wird zusammen mit Ruß C aus einem Rußspeicher 30 und Wasser in einer Kohle-Vergasungseinheit 20 zu Synthesegas umgesetzt, welches Kohlendioxid CO₂, Kohlenmonoxid CO und Wasserstoff H₂ enthält. Alternativ ist es möglich, den Ruß C aus dem Rußspeicher 30 unter Zufuhr von Sauersoff O₂ zu CO₂ zu verbrennen. Sowohl bei der Verbrennung, als auch beim Umsetzen zu Synthesegas wird Wärme frei, die zum Vorwärmen des Wassers H₂O genutzt wird, welches der Elektrolyse 18 zugeführt wird. Ebenso kann die Wärme zur Stromerzeugung verwendet werden, wobei der erzeugte elektrische Strom über eine Verbindung 36 ebenfalls für die Elektrolyse 18 genutzt werden kann.

Das aus der Kohle-Vergasungseinheit 20 entstandene Synthesegas bzw. Kohlendioxid wird anschließend zusammen mit dem Wasserstoff H₂ aus der Elektrolyse 18 in die Methanisierung 22 eingegeben. Dort wird in einer Sabatier-Reaktion bzw. einer Hydrierungsreaktion Methangas erzeugt. Dieses wird komprimiert und von verbleibendendem Kohlendioxid befreit in das öffentliche Gasnetz 24 eingespeist, welches als Gasspeicher 26 dient.

Wird elektrische Energie benötigt, weil gerade keine Windenergie 12 oder Sonnenenergie 14 zur Verfügung steht, wird Methangas aus dem Gasnetz 24 entnommen und in die Methan-Strom-Umwandlungsanlage 92 eingegeben. Dort wird das Methangas durch einen Wasserstoffgenerator 28 in Wasserstoff H₂ und Ruß C aufgespalten. Der Ruß C wird dem Speicher 30 zugeführt, wo er für eine erneute Methanumwandlung zur Verfügung steht. Der Wasserstoff H₂ aus dem Wasserstoffgenerator 28 wird nun in ein Gaskraftwerk 32 eingegeben, welches diesen verbrennt und elektrischen Strom herstellt. Der erzeugte elektrische Strom wird nun wieder in das Stromnetz 10 eingespeist.

Alternativ oder zusätzlich kann über einen weiteren Wasserstoffgenerator 29 Wasserstoff 34 für die Betankung von wasserstoffbetriebenen Fahrzeugen oder für andere Zwecke, wie beispielsweise zum Heizen, erzeugt werden.

Der eingesetzte Kohlenstoff wird hierbei vollständig im Kreislauf geführt, wobei dieser in fester Form zwischen der Methan-Strom-Umwandlungsanlage 92, dem Kohlenstoffspeicher 30 und der Strom-Methan-Umwandlungsanlage 90 transportiert wird. Eine aufwendige Abscheidung von Kohlendioxid aus Verbrennungsabgasen sowie der Transport von gasförmigem Kohlendioxid werden somit vermieden.

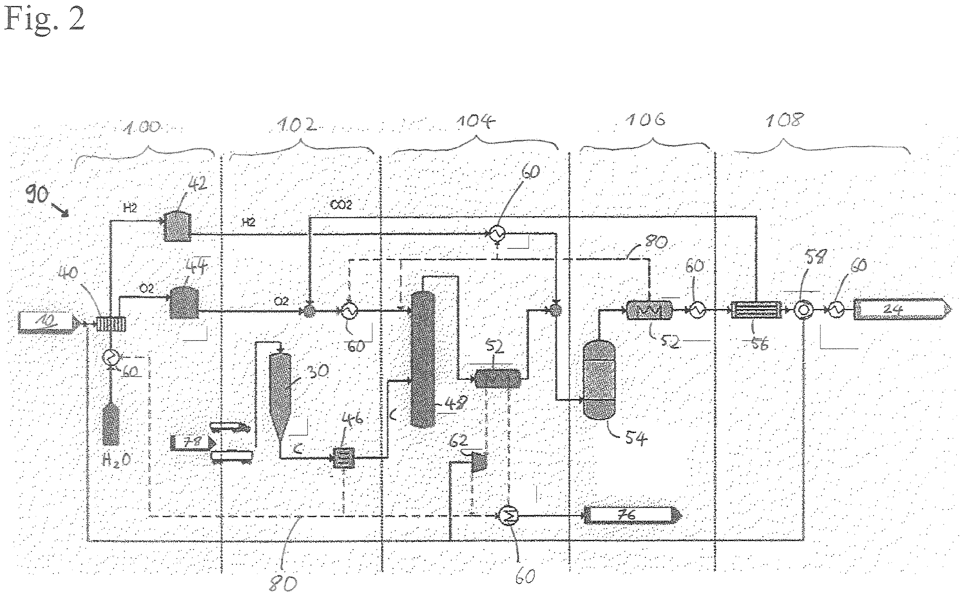

Figur 2 zeigt ein Schema einer ersten Ausführungsform einer Strom-Methan-Umwandlungsanlage.

In Figur 2 ist eine Strom-Methan-Umwandlungsanlage 90 schematisch dargestellt. Die Anlage umfasst fünf Abschnitte. Im ersten Abschnitt 100 wird die Elektrolyse durchgeführt. Elektrischer Strom wird aus dem Stromnetz 10 entnommen und einem Wasser-Elektrolyseur 40 zugeführt. Der Elektrolyseur 40 spaltet Wasser H₂O, welches mit Hilfe von Prozessdampf in einem Wärmetauscher 60 auf ca. 90°C erwärmt wird, in Wasserstoff H₂ und Sauerstoff O₂ auf. Die Elektrolyseprodukte werden jeweils in einem Wasserstoffbehälter 42 und einem Sauerstoffbehälter 44 zwischengelagert. Sind ausreichende Mengen an Wasserstoff und Sauerstoff vorhanden, wird mit der Methanerzeugung begonnen.

Dazu wird im zweiten Abschnitt 102 Ruß C an einer Anlieferungsstelle 78 entnommen und in einen Kohlenstoffspeicher 30 eingegeben. Vom Kohlenstoffspeicher 30 aus wird der Ruß C entnommen und einem Trockner 46 zugeführt. Der Ruß C wird zusammen mit Sauerstoff O₂ aus dem Sauerstoffbehälter 44 in eine Kohlenstoffvergasungseinheit 48 eingegeben. Über einen Wärmetauscher 60 im zweiten Abschnitt 102 wird dabei der Sauerstoff vorgewärmt.

Im dritten Abschnitt 104 wird der Ruß in Synthesegas umgesetzt. Der Ruß und der vorgewärmte Sauerstoff reagieren bei einer Temperatur zwischen 900 und 1800 °C, bevorzugt zwischen 1200 und 1800 °C, unter Einwirkung von Wasserdampf in der Kohlenstoffvergasungseinheit 48 zu Synthesegas. Besonders bevorzugt beträgt die Temperatur bei der Umsetzung ca. 1500°C. Die bei der Reaktion des Rußes zum Synthesegas freiwerdende Wärme kann mit einem Dampferzeuger 52 entnommen werden und als Prozessdampf genutzt werden. In der in Figur 2 dargestellten Ausführungsform wird der Prozessdampf über eine Wärmeleitung 80 zu einem Wärmetauscher 60 geführt, der Wasser vor dem Eintritt in den Elektrolyseur 40 erwärmt. Ebenfalls ist es möglich, einen Teil des Prozessdampfes zur Stromerzeugung in einem Stromgengerator 62 zu verwenden. Der erzeugte elektrische Strom kann in das Stromnetz 10 eingespeist werden und zur Elektrolyse eingesetzt werden.

Nach der Vergasung des Kohlenstoffs wird dem Synthesegas der Wasserstoff aus der Elektrolyse zugegeben, wobei ein für die Hydrierungsreaktion optimales Verhältnis zwischen Kohlenmonoxid aus dem Synthesegas und Wasserstoff eingestellt wird. Optimal ist hierbei ein stöchiometrisches (1:3) bis leicht überstöchiometrisches Verhältnis bezogen auf Wasserstoff.

Das Gasgemisch strömt nun in eine Hydrierungsanlage 54 im vierten Abschnitt 106 ein, wo das Synthesegas auf einem Katalysator in Methan umgesetzt wird, wobei CO + 3H₂ zu CH₄ + H₂O reagiert. Wiederum kann die bei der Hydrierungsreaktion freiwerdende Wärme mit einem Dampferzeuger 52 entnommen werden und als Prozessdampf genutzt werden. In der dargestellten Ausführungsform wird die Wärme über eine Wärmeleitung 80 zu einem Wärmetauscher 60 geführt, der den Sauerstoff vor dem Eintritt in die Kohlenstoffvergasungseinheit 48 erwärmt. Ein weiterer Wärmetauscher 60 wärmt den Wasserstoff vor dem Vermischen mit dem Synthesegas vor. Über einen Wärmetauscher 60 wird das Methangas abgekühlt.

Im letzten Abschnitt 106 wird das Methangas durch eine Kohlendioxidabtrennung 56 geleitet. In der Kohlendioxidabtrennung 56 wird im Methangas verbliebenes CO₂ beispielsweise mit Hilfe von Polyimid-Hohlfaser-Membranen abgeschiedenen und zurück in den Methanisierungsprozess geführt. Das CO₂ wird zusammen mit dem Sauerstoff O₂ wieder der Kohlenstoffvergasung 48 zugeführt. Das Methangas wird anschließend über einen Methankompressor 58 komprimiert. Dabei entstehende Wärme wird über einen weiteren Wärmetauscher 60 abgeführt, bevor das Methangas in das öffentliche Gasnetz 24 eingespeist wird. Das Gasnetz 24 dient hier zum einen als Transportmedium und zum anderen als Gasspeicher.

Prozesswärme, die nicht zur Vorwärmung des Wassers, des Wasserstoffs oder des Sauerstoffs benötigt wird, kann über einen weiteren Wärmetauscher 60 in ein Fernwärmenetz 76 eingespeist werden und weiter genutzt werden.

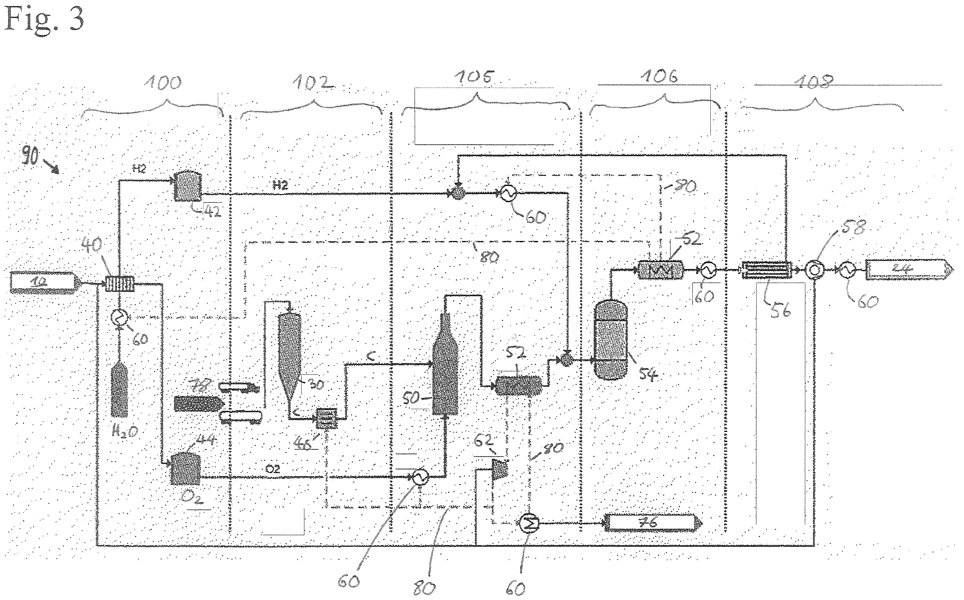

Figur 3 zeigt ein Schema einer weiteren Ausführungsform einer Strom-Methan-Umwandlungsanlage.

In Figur 3 ist eine zweite Ausführungsform einer Strom-Methan-Umwandlungsanlage 90 schematisch dargestellt. Die Anlage umfasst wiederum fünf Abschnitte. Im ersten Abschnitt 100 wird die Elektrolyse durchgeführt. Elektrischer Strom wird aus dem Stromnetz 10 entnommen und einem Wasser-Elektrolyseur 40 zugeführt. Der Elektrolyseur 40 spaltet Wasser H₂O, welches mit Hilfe von Prozessdampf in einem Wärmetauscher 60 auf ca. 90°C erwärmt wird, in Wasserstoff H₂ und Sauerstoff O₂ auf. Die Elektrolyseprodukte werden jeweils in einem Wasserstoffbehälter 42 und einem Sauerstoffbehälter 44 zwischengelagert. Sind ausreichende Mengen an Wasserstoff und Sauerstoff vorhanden, wird mit der Methanerzeugung begonnen.

Dazu wird im zweiten Abschnitt 102 Ruß C an einer Anlieferungsstelle 78 entnommen und in einen Kohlenstoffspeicher 30 eingegeben. Vom Kohlenstoffspeicher 30 aus wird der Ruß C entnommen und einem Trockner 46 zugeführt. Der Ruß C wird zusammen mit Sauerstoff O₂ aus dem Sauerstoffbehälter 44 in eine Kohlenstoffvergasungseinheit 48 eingegeben.

Im dritten Abschnitt 105 wird der Ruß zusammen mit dem Sauerstoff O₂ aus der Elektrolyse zu Kohlendioxid CO₂ verbrannt. Über einen Wärmetauscher 60 wird dabei der Sauerstoff vorgewärmt. Die bei der Verbrennung des Rußes C freiwerdende Wärme kann mit einem Dampferzeuger 52 entnommen werden und als Prozessdampf genutzt werden. Über eine Wärmeleitung 80 kann der Prozessdampf zum Rußtrockner 46 geführt werden. Ebenfalls ist es möglich, einen Teil des Prozessdampfes zur Stromerzeugung in einem Stromgenerator 62 zu verwenden. Der erzeugte elektrische Strom kann in das Stromnetz 10 eingespeist werden und zur Elektrolyse eingesetzt werden.

Dem erzeugten CO₂ wird nun im vierten Abschnitt 106 der bei der Elektrolyse gewonnene Wasserstoff zugegeben, wobei ein für die Sabatier-Reaktion optimales Verhältnis zwischen Kohlendioxid und Wasserstoff eingestellt wird. Optimal ist ein stöchiometrisches (1:4) bis leicht überstöchiometrisches Verhältnis bezogen auf Wasserstoff. Das Gasgemisch strömt nun in eine Hydrierungsanlage 54 ein, wo es auf einem Katalysator zu Methan umgesetzt wird. Die bei der Sabatier-Reaktion CO₂ + 4H₂ = CH4 + 2H₂O freiwerdende Wärme kann ebenfalls mit einem Dampferzeuger 56 entnommen werden und als Prozessdampf genutzt werden. In der in Figur 3 dargestellten Ausführungsform wird der Prozessdampf über eine Wärmeleitung 80 zu dem Wärmetauscher 60 geführt, um Wasser vor dem Eintritt in den Elektrolyseur 40 vorzuwärmen. Über einen weiteren Wärmetauscher 60 wird der Wasserstoff vor dem Vermischen mit dem Kohlendioxid erwärmt.

Im letzten Abschnitt 106 wird das Methangas durch eine Kohlendioxidabtrennung 56 geleitet. In der Kohlendioxidabtrennung 56 wird im Methangas verbliebenes CO₂ beispielsweise mit Hilfe von Polyimid-Hohlfaser-Membranen abgeschiedenen und zurück in den Methanisierungsprozess geführt. Das CO₂ wird zusammen mit dem Wasserstoff H₂ aus der Elektrolyse wieder der Hydrierungsanlage 54 zugeführt. Das Methangas wird anschließend über einen Methankompressor 58 komprimiert. Dabei entstehende Wärme wird über einen weiteren Wärmetauscher 60 abgeführt, bevor das Methangas in das öffentliche Gasnetz 24 eingespeist wird.

Prozesswärme, die nicht zur Vorwärmung des Wassers, des Wasserstoffs oder des Sauerstoffs benötigt wird, kann über einen weiteren Wärmetauscher 60 in ein Fernwärmenetz 76 eingespeist werden und weiter genutzt werden.

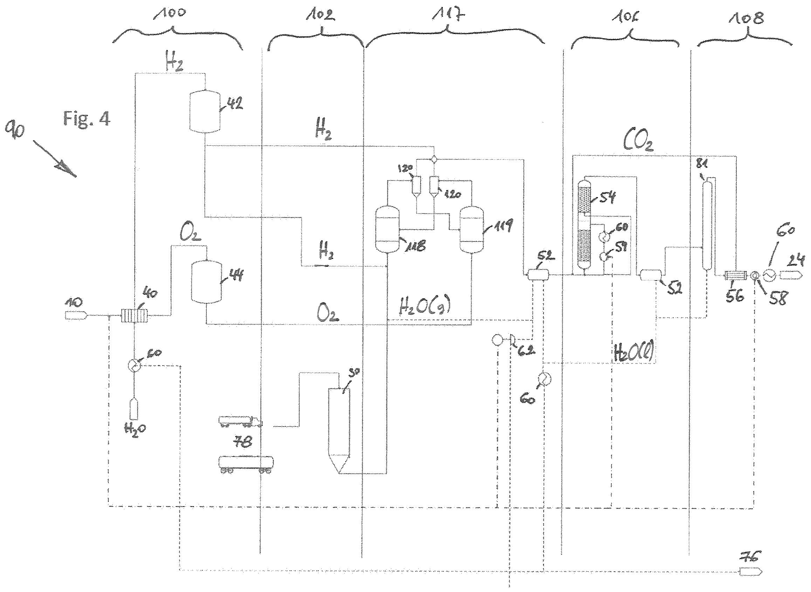

Figur 4 zeigt ein Schema einer dritten Ausführungsform einer Strom-Methan-Umwandlungsanlage.

Die in Figur 4 schematisch dargestellte Strom-Methan-Umwandlungsanlage 90 kann wie die beiden vorangegangen Ausführungsformen in fünf Abschnitte unterteilt werden. Im ersten Abschnitt 100 wird die Elektrolyse durchgeführt. Die Betriebsparameter und technische Gestaltung unterscheiden sich nicht von den beiden zuvor beschriebenen Ausführungsbeispielen.

Im Abschnitt 102 wird die Speicherstelle für Ruß dargestellt. Ruß C wird an einer Anlieferungsstelle 78 entnommen und in einen Kohlenstoffspeicher 30 eingegeben. Von dort aus wird der Ruß C entnommen und zum Hydrovergasungsanlage 118 transportiert. Trocknung des Rußes C ist nicht erforderlich.

Im dritten Abschnitt 117 wird Ruß zusammen mit Wasserstoff und Wasserdampf einem Hydrovergasungsapparat 118 zugeführt. Mit dem Gasgemisch wird dort auf eine Temperatur im Bereich von 700 bis 800°C vorgewärmter Sand fluidisiert. Der Sand stellt ein Feststoffbett dar. Im Apparat herrscht ein Druck im Bereich von 2,0 bis 3,0 MPa (20 bis 30 Bar). Das H₂-H₂O-Ruß-Gemisch wird zu Methan CH₄ und Kohlendioxid CO₂ umgesetzt. Das Verhältnis der Produkte CH₄ : CO₂ beträgt ca. 1 : 1. Die Produktgase und ein Teil des Sandbetts werden aus dem Apparat ausgetragen. Sand wird im Zyklon 120 abgetrennt und dem Regenerator 119 zugeführt. Da Ruß im Reaktor 118 nicht vollständig umgesetzt wird, wird er zusammen mit Sand dem Regenerator 119 zugeführt. Im Regenerator 119 wird der Ruß unter Einwirkung vom Sauerstoff aus dem Vorratsbehälter 44 verbrannt. Hierbei wird Sand ebenfalls fluidisiert. Regenerierter Sand wird in einem weiteren Zyklon 120 abgetrennt und in den Reaktor 118 zurückgeführt.

Die Verbrennungsgase werden mit Reaktionsprodukten und zusätzlichem Wasserstoff aus dem Behälter 42 vermischt, so dass ein optimales Verhältnis zwischen Kohlendioxid und Wasserstoff von 1 : 4 eingestellt wird. Das Gemisch wird auf die für die nachfolgende Methanisierung optimale Temperatur abgekühlt. In dieser Ausführungsform wird die Abwärme für die Dampferzeugung und Stromherstellung verwendet. Strom wird intern für das Betreiben der Kompressoren 58 aber auch zur Wasserelektrolyse 40 angewandt.

Im vierten Abschnitt 106 erfolgt in einer Hydrierungsanlage 54 die Umsetzung des Kohlendioxids zum Methan auf einem Katalysator. Der Katalysator kann in mehrere, beispielhaft zwei, Abschnitte unterteilt werden. Zwischen den Abschnitten wird die Reaktionswärme abgeführt, um die Ausbeute zu erhöhen. Alternativ kann zum gleichen Zweck eine Reaktorenkaskade verwendet werden. Ein Teil des Reaktionsgasgemisches wird zu diesem Zweck mittels eines Gebläses 59 im Kreis geführt. Der im nachgeschalteten Dampferzeuger 52 produzierte Wasserdampf H₂O (g) wird verstromt und zur Elektrolyse im Elektrolyseur 40 benutzt.

Im letzten Abschnitt 108 wird zunächst das Nebenprodukt Wasser H₂O (I) in einem Abscheider 81 durch Kondensation vom Gasgemisch abgetrennt. Anschließend wird wie zuvor zu der ersten und zweiten Ausführungsform beschrieben das Methangas zur Einspeisung in das öffentliche Gasnetz 24 vorbereitet.

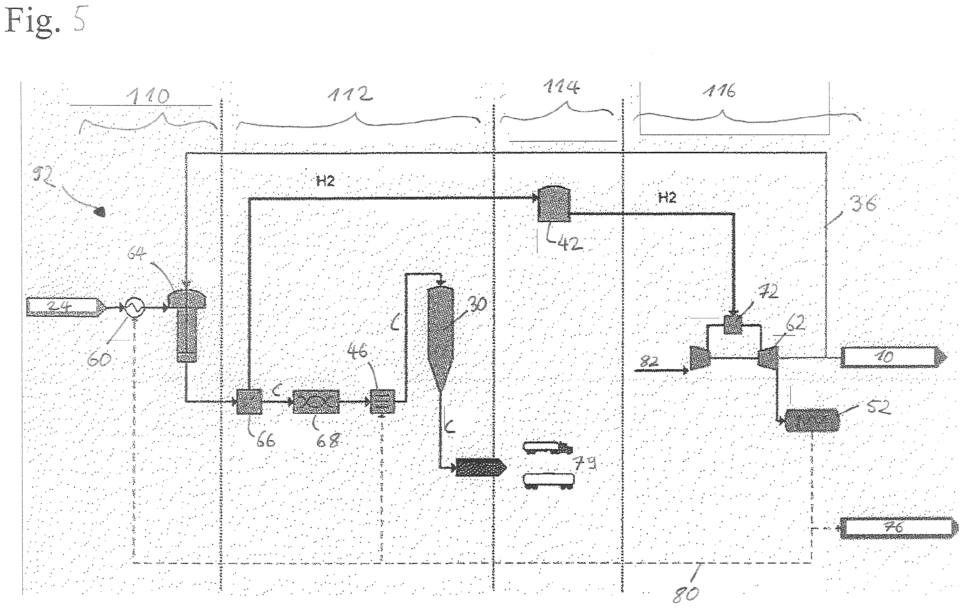

Figur 5 zeigt ein Schema einer Methan-Strom-Umwandlungsanlage.

In Figur 5 ist eine Methan-Strom-Umwandlungsanlage 92 dargestellt. Im ersten Abschnitt 110 der Anlage wird Methangas aus dem Gasnetz 24 entnommen. Über einen Wärmetauscher 60 wird das Methangas vorgewärmt und anschließend in einen Plasma-Wasserstoffgenerator 64 eingegeben. Dort wird mit Hilfe von Mikrowellenstrahlung ein Plasma erzeugt, welches bei einer Temperatur zwischen ca. 400 und 600 °C das Methangas in Kohlenstoff (Ruß) und Wasserstoff H₂ aufspaltet.

Wasserstoff und Kohlenstoff werden aus dem Plasma-Wasserstoffgenerator 64 entnommen und im zweiten Abschnitt 112 an einem Rußfilter 66 voneinander getrennt. Der Ruß wird in einer Granulationseinrichtung 68 agglomeriert. Diese kann beispielsweise als Trommel-Granulator ausgeführt sein. Anschließend wird der Ruß in einem Trockner 46 getrocknet, bevor er in einen Kohlenstoffspeicher 30 transportiert wird. Von dort aus kann der Ruß über eine Verladevorrichtung 79 in einem geeigneten Transportbehälter abtransportiert werden. Der Rücktransport zur Strom-Methan-Umwandlungsanlage kann beispielsweise über LKW oder Eisenbahnwagons erfolgen.

Der abgetrennte Wasserstoff wird im dritten Abschnitt 114 in einem Wasserstoffbehälter 42 zwischengelagert, bevor er im vierten Abschnitt 116 in Strom umgesetzt wird. Dazu wird der Wasserstoff H₂ mit Luft 82 vermischt und anschließend in einer Wasserstoffturbine 72 verbrannt. Ein Stromgenerator 62 erzeugt aus den heißen Verbrennungsabgasen elektrischen Strom, der in das Stromnetz 10 eingespeist werden kann. Ein Teil der erzeugten elektrischen Energie wird von der Umwandlungsanlage selbst für den Betrieb des Plasma-Wasserstoffgenerators 64 benötigt und über die Stromleitung 36 geführt.

Die bei der Verbrennung des Wasserstoffs anfallende Wärme kann über einen Dampferzeuger 52 als Prozesswärme genutzt werden. Über eine Wärmeleitung 80 kann der erzeugte Dampf verwendet werden, um beispielsweise das Methangas vorzuheizen. Ebenfalls ist es möglich, einen Teil der Wärme in ein Fernwärmenetz 76 einzuspeisen.

Alternativ kann auch eine Methan-Strom-Umwandlungsanlage eingesetzt werden, die auf Basis eines zyklischen Bromierungs-Oxidations-Prozesses arbeitet. Dabei wird zunächst mit Hilfe des zyklischen Bromierungs-Oxidations-Prozesses Wärme hergestellt. Diese kann zur Stromerzeugung verwendet werden und/oder in ein Fernwärmenetz eingespeist werden.

In einer weiteren Ausführungsform kann anstelle des Plasma-Verfahrens zur Spaltung des Methans ein thermisches Verfahren wie z.B. das Gasruß-Degussa-Verfahren eingesetzt werden.

Die Erfindung ist nicht auf die hier beschriebenen Ausführungsbeispiele und die darin hervorgehobenen Aspekte beschränkt. Vielmehr sind innerhalb des durch die Ansprüche angegebenen Bereichs eine Vielzahl von Abwandlungen möglich, die im Rahmen fachmännischen Handelns liegen.

**Bezugszeichenliste**

| | |
|---|---|
| 10 | Stromnetz |
| 12 | Windenergie |
| 14 | Solarenergie |
| 16 | sonstige regenerative Energie |
| 18 | Wasserelektrolyse und H₂- und O₂- Speicher |
| 20 | Kohle-Vergasung / Kohleverbrennung und Kraft-Wärme-Kopplung |
| 22 | Methanisierung |
| 24 | Gasnetz |
| 26 | Gasspeicher |
| 28, 29 | H₂-Generator |
| 30 | Kohlespeicher |
| 32 | Gaskraftwerk, Brennstoffzellen, Kraft-Wärme-Kopplung |
| 36 | Stromleitung |
| 38 | Stromerzeugung |
| 40 | Wasser-Elektrolyseur |
| 42 | Wasserstoff-Behälter |
| 44 | Sauerstoff-Behälter |
| 46 | Ruß-Trocker |
| 48 | Kohlenstoffvergasung |
| 50 | Kohlenstoffverbrennung |
| 52 | Dampferzeuger |
| 54 | Hydrierungsanlage |
| 56 | CO₂-Abtrennung |
| 58 | Methankompressoren |
| 59 | Gebläse |
| 60 | Wärmetauscher |
| 62 | Stromgenerator |
| 64 | Plasma-Wasserstoffgenerator |
| 66 | Ruß-Filter |
| 68 | Ruß-Granulator |
| 70 | Ruß-Verladestation |
| 72 | Wasserstoff-Gasturbine |
| 74 | Stromgenerator |
| 76 | Fernwärmenetz |
| 78 | Anlieferung Ruß |
| 79 | Abtransport Ruß |
| 80 | Wärmeleitung |
| 81 | Abscheider |
| 82 | Luft |
| 90 | Strom-Methan-Umwandlungsanlage |
| 92 | Methan-Strom-Umwandlungsanlage |
| 100 | Elektrolyse |
| 102 | Ruß-Lagerung |
| 104 | Ruß-Vergasung |
| 105 | Ruß-Verbrennung |
| 106 | Methanisierung |
| 108 | Methan-Aufbereitung |
| 110 | Methanspaltung |
| 112 | Ruß-Behandlung |
| 114 | H₂-Speicher |
| 116 | Stromerzeugung |
| 117 | Ruß-Hydrovergasung |
| 118 | Hydrovergasungsanlage |
| 118 | Regenerator |
| 120 | Zyklon |
| H₂O | Wasser |
| O₂ | Sauerstoff |
| H₂ | Wasserstoff |
| CO | Kohlenmonoxid |
| CO₂ | Kohlendioxid |
| C | Kohlenstoff, Ruß |

## Patentansprüche

1. Verfahren zur Erzeugung von Methan unter Verwendung von elektrischer Energie und nachfolgender Energieerzeugung, umfassend die Schritte
a) Erzeugung von Methan aus Wasser und Ruß unter Verwendung von elektrischer Energie,
b) Speichern des Methans,
c) Spalten des Methans in Wasserstoff und Ruß, wobei der Wasserstoff zur Energieerzeugung verwendet wird oder,
Energieerzeugung durch Umsetzen des Methans in Ruß und Wasser in einer zyklischen Bromierungs-Oxidations-Prozess,
**dadurch gekennzeichnet, dass** der bei der Methanspaltung oder dem zyklischen Bromierungs-Oxidations-Prozess gemäß Schritt c) anfallende Ruß aufgefangen wird und beim erneuten Durchlaufen des Verfahrens für die Methanerzeugung in Schritt a) verwendet wird, so dass ein geschlossener Kohlenstoffkreislauf entsteht.

2. Verfahren nach Anspruch 1, **dadurch gekennzeichnet, dass** die Erzeugung des Methans mittels einer Sabatier-Reaktion oder mittels einer Hydrierungsreaktion erfolgt.

3. Verfahren nach Anspruch 2, **dadurch gekennzeichnet, dass** der bei der Sabatier-Reaktion oder bei der Hydrierungsreaktion benötigte Wasserstoff mittels Elektrolyse von Wasser gewonnen werden.

4. Verfahren nach Anspruch 2 oder 3, **dadurch gekennzeichnet, dass** der Ruß für die Durchführung der Sabatier-Reaktion zu Kohlendioxid verbrannt wird oder der Ruß unter Einwirkung von Sauerstoff und Wasserdampf in Synthesegas umgesetzt wird, wobei der Sauerstoff mittels Elektrolyse von Wasser gewonnen wird.

5. Verfahren nach einem der Ansprüche 1 bis 4, **dadurch gekennzeichnet, dass** die Speicherung des Methans durch Einspeisen in ein Gasnetz (24) erfolgt.

6. Verfahren nach einem der Ansprüche 1 bis 5, **dadurch gekennzeichnet, dass** die Spaltung des Methans und die Energieerzeugung gemäß Schritt c) an einem anderen Ort als die Erzeugung des Methans gemäß Schritt a) durchgeführt werden.

7. Verfahren nach einem der Ansprüche 1 bis 6, **dadurch gekennzeichnet, dass** zur Energieerzeugung gemäß Schritt c) des Verfahrens der erhaltene Wasserstoff in einer Brennstoffzelle in elektrische Energie umgesetzt wird, der Wasserstoff zum Heizen verwendet wird, der Wasserstoff zum Kühlen verwendet wird, der Wasserstoff als Treibstoff für ein Transportmittel dient oder der Wasserstoff für eine Kombination mindestens zweier der genannten Zwecke verwendet wird.

8. Verfahren nach einem der Ansprüche 1 bis 7, **dadurch gekennzeichnet, dass** bei der Methanerzeugung gemäß Schritt a) und/oder bei der Energieerzeugung gemäß Schritt c) anfallende Wärme in ein Fernwärmenetz (76) eingespeist wird.

9. Verfahren nach einem der Ansprüche 1 bis 8, **dadurch gekennzeichnet, dass** die im Schritt a) eingesetzte elektrische Energie regenerativ erzeugt wird.

10. Verfahren nach Anspruch 9, **dadurch gekennzeichnet, dass** die elektrische Energie mittels Windkraft (12) oder Solaranlagen (14) erzeugt wird.

## Claims

1. Method for the production of methane using electric energy and subsequent energy generation, which comprises the steps
a) production of methane from water and soot using electric energy,
b) storage of the methane,
c) dissociation of the methane into hydrogen and soot, with the hydrogen being used for energy generation or,
energy generation by conversion of the methane into soot and water in a cyclic bromination-oxidation process,
**characterized in that** the soot formed in the dissociation of methane or in the cyclic bromination-oxidation process in step c) is collected and, in a renewed pass through the method, is used for methane production in step a), so that a closed carbon circuit is formed.

2. Method according to Claim 1, **characterized in that** the production of the methane is carried out by means of a Sabatier reaction or by means of a hydrogenation reaction.

3. Method according to Claim 2, **characterized in that** the hydrogen required in the Sabatier reaction or in the hydrogenation reaction is obtained from water by means of electrolysis.

4. Method according to Claim 2 or 3, **characterized in that** the soot for carrying out the Sabatier reaction is burnt to form carbon dioxide or the soot is converted in the presence of oxygen and steam into synthesis gas, with the oxygen being obtained by means of electrolysis of water.

5. Method according to any of Claims 1 to 4, **characterized in that** the storage of the methane is effected by feeding into a gas grid (24).

6. Method according to any of Claims 1 to 5, **characterized in that** the dissociation of the methane and the energy generation according to step c) are carried out at a different place than the production of the methane according to step a).

7. Method according to any of Claims 1 to 6, **characterized in that**, for the energy generation according to step c) of the method, the hydrogen obtained is converted in a fuel cell into electric energy, the hydrogen is used for heating, the hydrogen is used for cooling, the hydrogen is used as fuel for a means of transport or the hydrogen is used for a combination of at least two of the purposes mentioned.

8. Method according to any of Claims 1 to 7, **characterized in that** heat arising in the production of methane according to step a) and/or in the energy generation according to step c) is fed into a district heating network (76).

9. Method according to any of Claims 1 to 8, **characterized in that** the electric energy used in step a) is generated using renewable resources.

10. Method according to Claim 9, **characterized in that** the electric energy is generated by means of wind power (12) or solar plants (14).

## Revendications

1. Procédé pour la production de méthane avec utilisation d'énergie électrique et production consécutive d'énergie, comprenant les étapes de
a) production de méthane à partir d'eau et de suie avec utilisation d'énergie électrique,
b) accumulation du méthane,
c) dissociation du méthane en hydrogène et en suie, l'hydrogène étant utilisé pour la production d'énergie ou production d'énergie par transformation du méthane en suie et eau dans un procédé de bromuration-oxydation cyclique,
**caractérisé en ce que** la suie obtenue lors de la dissociation du méthane ou du procédé de bromuration-oxydation cyclique selon l'étape c) est récupérée et utilisée lors d'un nouveau déroulement du procédé pour la production de méthane dans l'étape a), de telle sorte qu'un circuit fermé de carbone se forme.

2. Procédé selon la revendication 1, **caractérisé en ce que** la production du méthane a lieu via une réaction de Sabatier ou via une réaction d'hydrogénation.

3. Procédé selon la revendication 2, **caractérisé en ce que** l'hydrogène nécessaire lors de la réaction de Sabatier ou de la réaction d'hydrogénation est obtenue par électrolyse de l'eau.

4. Procédé selon la revendication 2 ou 3, **caractérisé en ce que** la suie pour la réalisation de la réaction de Sabatier est brûlée en dioxyde de carbone ou la suie est transformée sous action d'oxygène et de vapeur d'eau en gaz de synthèse, l'oxygène étant obtenu par électrolyse de l'eau.

5. Procédé selon l'une quelconque des revendications 1 à 4, **caractérisé en ce que** l'accumulation du méthane est réalisée par injection dans un réseau de gaz (24).

6. Procédé selon l'une quelconque des revendications 1 à 5, **caractérisé en ce que** la dissociation du méthane et la production d'énergie selon l'étape c) sont réalisées en un autre site que celui de la production du méthane selon l'étape a).

7. Procédé selon l'une quelconque des revendications 1 à 6, **caractérisé en ce que** pour la production d'énergie selon l'étape c) du procédé, l'hydrogène obtenu est transformé en énergie électrique dans une pile à combustible, l'hydrogène est utilisé pour le chauffage, l'hydrogène est utilisé pour le refroidissement, l'hydrogène sert de carburant pour un moyen de transport ou l'hydrogène est utilisé pour une combinaison d'au moins deux des objectifs mentionnés.

8. Procédé selon l'une quelconque des revendications 1 à 7, **caractérisé en ce que** la chaleur obtenue lors de la production de méthane selon l'étape a) et/ou lors de la production d'énergie selon de l'étape c) est injectée dans un réseau de chauffage collectif (76).

9. Procédé selon l'une quelconque des revendications 1 à 8, **caractérisé en ce que** l'énergie électrique utilisée dans l'étape a) est produite par régénération.

10. Procédé selon la revendication 9, **caractérisé en ce que** l'énergie électrique est produite par une installation éolienne (12) ou solaire (14).
